# EUROPEAN PATENT APPLICATION

(11) **EP 4 714 375 A1**
(43) Date of publication of application: **25.03.2026**
(21) Application number: 24807549.1
(22) Date of filing: 14.05.2024
(51) Int. Cl.: A61B 17/70, A61B 17/88, A61B 17/00

(54) **PEDICLE SCREW ASSEMBLY**

(30) Priority: 18.05.2023 KR 20230064289; 12.12.2023 KR 20230179877
(71) Applicant: CG Bio Co., Ltd., Seoul 04349 (KR)
(72) Inventor: JUNG, Ui Su, Seoul 05393 (KR); CHO, Samuel, Englewood Cliffs,, New Jersey 07632 (US); SON, Dong Min, Seongnam-si, Gyeonggi-do 13164 (KR)
(74) Representative: Laine IP Oy
(86) International application number: PCT/KR2024/006604
(87) International publication number: WO 2024/237678

(57) **Abstract**

Disclosed is a pedicle screw assembly. A pedicle screw assembly according to one aspect of the present invention comprises: a screw member coupled to a pedicle; a rod support member for rotatably supporting the screw member; a driver support member extending in one direction and having one side coupled to the rod support member; and a reinforcing member coupled to the other side of the driver support member and configured to press the driver support member radially inward. The driver support member comprises: a driver support body extending in the one direction and having one side in the extension direction coupled to the rod support member; and a reinforcing member coupling part which is continuous with the other side of the driver support body in the extension direction, extends in the one direction, and is coupled to the reinforcing member.

## Description

### [TECHNICAL FIELD]

The present disclosure relates to a pedicle screw assembly, and more particularly, to a pedicle screw assembly having a structure capable of stably maintaining a coupling state between a rod and a rod support member.

### [BACKGROUND ART]

A pedicle screw, also referred to as a pedicle screw implant (hereinafter referred to as "pedicle screw"), is a device fixed to a spine. The pedicle screw is widely used to restore a deformed spine to its normal alignment. Specifically, after a plurality of pedicle screws are coupled to pedicles, a rod or similar component is inserted into the coupled pedicle screws to apply an external force, thereby allowing the spine to be restored back to its normal state.

The pedicle screws penetrate the skin and muscles covering the spine to access the spine. Therefore, in order to insert and fix the pedicle screw into the spine, an invasive procedure, that is, an incision at the surgical site, must be performed beforehand.

However, as the size of the invasive site increases, the difficulty of the surgery also rises, and the patient may experience greater pain. In addition, if the size of the invasive site becomes excessively large, there is an increased risk of secondary infections caused by bacteria and other pathogens.

Therefore, recently, various approaches have been actively discussed to minimize the size of the invasive site while enabling the insertion of the pedicle screw.

In addition, in the case of the pedicle screw, some components for supporting a screw coupled to the spine remain indwelling in the body. That is, a driver for coupling the screw to the spine is inserted separately from the pedicle screw. For example, the driver is inserted through a component configured to support the pedicle screw.

In this case, as the length of the pedicle screw and the driver increases, there is a risk that a fine movement outside the body acts as a large movement inside the body. Therefore, it is also essential to stably support the driver coupled to the pedicle screw during the procedure of the pedicle screw.

Korean Patent No. 10-1814838 discloses a system and method for pedicle screw stabilization of the spine. Specifically, it discloses a system and method for pedicle screw stabilization of the spine, comprising one or more guide members connected to a screw member, capable of guiding a rod implant or instrument to a desired site within the patient.

However, the system and method for pedicle screw stabilization of the spine disclosed in the prior art are configured to support the rod using a separate guide member coupled with the screw threads. In particular, to connect multiple screw threads using a single rod, the guide members disclosed in the prior art are arranged to intersect one another within the patient's body.

In other words, the system and method for pedicle screw stabilization of the spine disclosed in the prior art provide only a means to guide the rod implant and instrument to a desired site within the patient, and do not provide a means to minimize the invasive site.

Korean Patent Laid-Open Publication No. 10-2008-0022767 discloses a device and method for manufacturing a rod for pedicle fixation, and a rod manufactured using the device. Specifically, it discloses a device and method for manufacturing a rod for pedicle fixation, and a rod manufactured using the device, which includes position moving means capable of simultaneous movement along the X-axis and Y-axis directions, including a servo motor, enabling accurate procedures to be performed on a desired site.

However, the manufacturing device and method, and the rod manufactured using the device disclosed in the prior art require additional mechanical means. Further, the set screw disclosed in the prior art is formed to have an excessive volume compared to a conventional pedicle screw. Therefore, to utilize the prior art, the invasive site would in fact need to be enlarged.

Korean Patent No. 10-1814838 (2018.01.03.)

Korean Patent Laid-Open No. 10-2008-0022767 (2008.03.12.)

### [DISCLOSURE]

### [TECHNICAL PROBLEM]

An object of the present disclosure is to solve the above problems and to provide a pedicle screw assembly having a structure capable of minimizing the invasive site.

Another object of the present disclosure is to provide a pedicle screw assembly having a structure in which the coupling state between the pedicle screw and another component can be stably maintained.

Yet another object of the present disclosure is to provide a pedicle screw assembly having a structure in which each component can be easily and robustly coupled.

Still another object of the present disclosure is to provide a pedicle screw assembly having a structure in which the coupling between an additional component for adjusting the pedicle screw and another component can be stably maintained.

The technical problems of the present disclosure are not limited to the above-mentioned problems, and other problems not mentioned will be clearly understood by those skilled in the art from the following description.

### [TECHNICAL SOLUTION]

According to an aspect of the present disclosure, a pedicle screw assembly is provided, including a screw member configured to be coupled to a pedicle; a rod support member configured to rotatably support the screw member; a driver support member extending in one direction, one side of the driver support member being coupled to the rod support member; a reinforcing member coupled to a different side of the driver support member, the reinforcing member being configured to press the driver support member radially inward, wherein the driver support member includes a driver support body extending in the one direction, one side of the driver support body in the extending direction being coupled to the rod support member; and a reinforcing-member coupler extending in the one direction, the reinforcing-member coupler being continuous with a different side of the driver support body in the extending direction and being coupled to the reinforcing member.

In this case, a pedicle screw assembly may be provided, wherein the reinforcing member includes a reinforcing body configured to surround the reinforcing-member coupling portion and to be coupled to the driver support member; and a support-member coupling space formed inside the reinforcing body, the support-member coupling space being open on one side facing the reinforcing member so as to detachably receive the reinforcing-member coupler.

In addition, a pedicle screw assembly may be provided, wherein the driver support body includes a pair of driver support bodies, the pair being spaced apart from each other in a different direction; the reinforcing-member coupler includes a pair of reinforcing-member couplers, the pair being respectively continuous with the pair of driver support bodies; and the support-member coupling space includes a pair of support-member coupling spaces, the pair being spaced apart from each other in the different direction and respectively receiving the pair of reinforcing-member couplers.

In this case, a pedicle screw assembly may be provided, wherein the reinforcing-member coupler includes a reinforcing-member receiving groove, the reinforcing-member receiving groove being recessed on an outer surface of the reinforcing-member coupler, and wherein reinforcing member includes a support-member coupling protrusion protruding from an inner surface surrounding the support-member coupling space so as to be received in the reinforcing-member receiving groove.

In addition, a pedicle screw assembly may be provided, wherein the reinforcing member is divided into one portion positioned inward and a different portion positioned outward along a radial direction with respect to the support-member coupling space, and wherein the different portion of the reinforcing member includes a slitted groove, the slitted groove extending along a height direction of the reinforcing member and penetrating through a thickness direction of the different portion.

In this case, a pedicle screw assembly may be provided, wherein the reinforcing member includes a pair of slitted grooves, the pair being spaced apart from each other along an outer circumference of the different portion of the reinforcing member, and wherein the reinforcing body includes a reinforcing wing positioned between the pair of slitted grooves, the reinforcing wing being configured to be elastically deformable radially outward when pressed by the reinforcing-member coupler.

In addition, a pedicle screw assembly may be provided, wherein the reinforcing member includes a support-member coupling protrusion, the support-member coupling protrusion protruding from an inner surface of the reinforcing wing so as to contact the reinforcing-member coupler.

In this case, a pedicle screw assembly may be provided, wherein a thread is formed on an outer circumferential surface of the reinforcing-member coupler, a thread is formed on an inner circumferential surface of the reinforcing member, such that the reinforcing-member coupler and the reinforcing member are screw-coupled to each other.

In addition, a pedicle screw assembly may be provided, wherein the rod support member includes a support-member coupler received in the driver support member and coupled thereto, and the driver support member includes a rod coupler positioned at one side of the driver support body and coupled to the support-member coupler.

In this case, a pedicle screw assembly may be provided, wherein the support member coupler and the rod coupler are hook-fitted to each other.

In addition, a pedicle screw assembly may be provided, wherein the rod coupler includes a movement groove configured to penetrate through a thickness direction of the driver support body and to extend along a longitudinal direction thereof; and a rod coupling projection disposed in the movement groove and extending in the same direction as the movement groove, one end thereof facing the rod support member, being formed as a free end, and the other end thereof facing the reinforcing member, being coupled to the driver support body.

In this case, a pedicle screw assembly may be provided, wherein the support-member coupler includes a coupling body extending in the same direction as the rod coupling projection; a coupling groove recessed at each edge in a width direction of the coupling body; and a coupling projection positioned at one end of the coupling body facing the driver support member, the coupling projection being configured to have a width greater than that of the coupling body.

In addition, a pedicle screw assembly may be provided, wherein the rod coupling projection includes a projection formed at one end thereof and protruding facing the rod support member, and the rod coupling projection is received in the coupling groove after the projection contacts the coupling projection and is elastically deformed outward.

In this case, a pedicle screw assembly may be provided, wherein the coupling projection includes a first projection surface positioned farthest from the driver support member and extending outward from an outer surface of the coupling body; a second projection surface continuous with the first projection surface and extending inward and inclined toward the driver support member; and a third projection surface continuous with the second projection surface and extending inward and inclined toward the driver support member, wherein the rod coupling projection is received in the coupling groove after being shape-deformed as it sequentially contacts the third projection surface and the second projection surface.

### [ADVANTAGEOUS EFFECTS]

According to the above configuration, the pedicle screw assembly according to the embodiment of the present disclosure can minimize the invasive site.

In addition, according to the above configuration, the pedicle screw assembly according to the embodiment of the present disclosure can stably maintain the coupling state between the pedicle screw and another component.

In addition, according to the above configuration, the pedicle screw assembly according to an embodiment of the present disclosure provides a pedicle screw assembly having a structure in which each component can be easily and robustly coupled.

In addition, according to the above configuration, the pedicle screw assembly according to the embodiment of the present disclosure can stably maintain the coupling between an additional component for adjusting the pedicle screw and another component.

It should be understood that the effects of the present disclosure are not limited to the above-described effects, and include all effects that can be inferred from the configuration of the invention described in the detailed description or the claims.

### [DESCRIPTION OF DRAWINGS]

FIG. 1 is a perspective view illustrating a pedicle screw assembly according to an embodiment of the present disclosure.
FIG. 2 is a side view illustrating the pedicle screw assembly of FIG. 1.
FIG. 3 is an exploded perspective view illustrating the configuration of the pedicle screw assembly of FIG. 1.
FIG. 4 is a perspective view illustrating a rod support member provided in the pedicle screw assembly of FIG. 1.
FIG. 5 is a plan view illustrating the rod support member of FIG. 4.
FIG. 6 is a side view illustrating the rod support member of FIG. 4.
FIG. 7 is a cross-sectional view A-A illustrating the rod support member of FIG. 4.
FIG. 8 is a perspective view illustrating the driver support member provided in the pedicle screw assembly of FIG. 1.
FIG. 9 is a plan view illustrating the driver support member of FIG. 8.
FIG. 10 is a side view illustrating the driver support member of FIG. 8.
FIG. 11 is a cross-sectional view taken along line B-B illustrating the driver support member of FIG. 8.
FIG. 12 is a perspective view illustrating the reinforcing member provided in the pedicle screw assembly of FIG. 1.
FIG. 13 is a front view illustrating the reinforcing member of FIG. 12.
FIG. 14 is a side cross-sectional view illustrating a process of coupling the rod support member and the driver support member.
FIG. 15 is a plan view illustrating a state in which the driver support member of FIG. 8 and the reinforcing member of FIG. 12 are coupled.

### [BEST EMBODIMENTS]

Hereinafter, embodiments of the present disclosure will be described in detail with reference to the accompanying drawings so that those of ordinary skill in the art may readily implement the present disclosure. The present disclosure may be embodied in various forms and is not limited to the embodiments set forth herein. For clarity, parts not related to the description are omitted from the drawings, and like reference numerals refer to like or similar elements throughout the specification.

The words and terms used in the present specification and claims should not be construed as being limited to their ordinary or dictionary meanings, but should be interpreted in accordance with the principle that an inventor may define terms and concepts to best describe the invention, and should be understood as having meanings and concepts consistent with the technical spirit of the present disclosure.

Therefore, the embodiments described in this specification and the configurations illustrated in the drawings correspond to a preferred embodiment of the present disclosure and do not represent all the technical ideas of the present disclosure; accordingly, various equivalents and modifications that can replace the corresponding configurations may exist as of the filing date of the present disclosure.

In the following description, to clearly explain the features of the present disclosure, descriptions of some components may be omitted.

As used in the following description, the term "communicating" means that one or more members are connected to allow fluid communication with each other. In an embodiment, the communicating may be formed by members such as conduits, pipes, or tubing. In the following description, "communicating" may be used to have the same meaning as one or more members being "fluidly connected" to each other.

As used in the following description, the term "fluid" refers to any form of matter that flows under external force and whose shape or volume can be deformed. In an embodiment, the fluid may be a liquid such as water or a gas such as air.

The terms "upper side", "lower side", "left side", "right side", "front side" and "rear side" as used in the following description will be understood with reference to the coordinate system shown throughout the accompanying drawings.

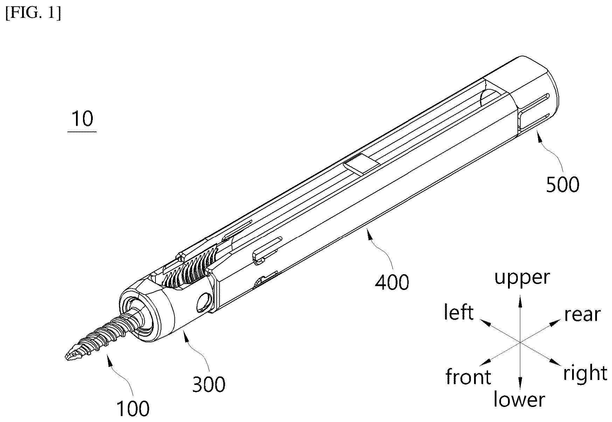

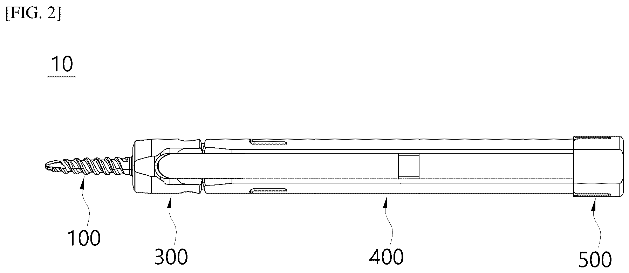

Referring to FIGS. 1 to 3, a pedicle screw assembly 10 according to an embodiment of the present disclosure is illustrated. The pedicle screw assembly 10 according to the embodiment of the present disclosure is configured to be coupled to a pedicle. A rod, which is separately provided, is coupled to the pedicle screw assembly 10 coupled to the pedicle, so that an external force can be applied to the pedicle through the rod.

For this purpose, a plurality of pedicle screw assemblies 10 may be provided. The plurality of pedicle screw assemblies 10 may be coupled to pedicles at different positions. The rod is coupled to each of the plurality of pedicle screw assemblies 10 so that the external force can be applied to the pedicles through the rod.

In the following description, it is assumed that the pedicle screw assembly 10 is coupled to the pedicle of a human body. Alternatively, the pedicle screw assembly 10 according to an embodiment of the present disclosure may be provided as any device assuming that it is inserted into the human body and coupled to a bone.

The pedicle screw assembly 10 according to an embodiment of the present disclosure may be formed to have a length in one direction, that is, in a front-rear direction in the illustrated embodiment. The pedicle screw assembly 10 may be inserted into the human body along its longitudinal direction, and one side in the longitudinal direction, that is, a front side in the illustrated embodiment, may be coupled to the pedicle. In addition, a configuration for forming a coupling with the pedicle may be coupled to the pedicle screw assembly 10 through the different side in its longitudinal direction.

In this case, the pedicle screw assembly 10 according to an embodiment of the present disclosure can stably support a configuration (for example, a driver) for forming a coupling with the pedicle, while minimizing an invasive region.

In the illustrated embodiment, the pedicle screw assembly 10 includes a screw member 100, a detachment-preventing member 200, a rod support member 300, a driver support member 400, and a reinforcing member 500.

The screw member 100 serves as a portion of the pedicle screw assembly 10 that is directly coupled to a pedicle. The screw member 100 may be fixedly coupled to the pedicle. In this case, the screw member 100 may be configured to be fixedly coupled to the pedicle by an external force, but may also be separable from the pedicle by the external force. In an embodiment, the screw member 100 may be provided in the form of a screw.

The screw member 100 constitutes one end in the longitudinal direction of the pedicle screw assembly 10. In the illustrated embodiment, the screw member 100 constitutes a front end of the pedicle screw assembly 10. The pedicle screw assembly 10 may be moved forward to be inserted into the human body and may be moved backward to be withdrawn from the human body.

The screw member 100 is coupled to a detachment-preventing member 200. Specifically, the screw member 100 may be coupled to the detachment-preventing member 200 so as to be rotatable about its longitudinal axis while being prevented from moving in the longitudinal direction. That is, the screw member 100 may be rotatably coupled to the detachment-preventing member 200.

As will be described later, the detachment-preventing member 200 may be rotatably coupled to the rod support member 300 about its longitudinal axis. Therefore, the screw member 100 may also be rotatably coupled to the rod support member 300.

The screw member 100 is coupled to the rod support member 300. The screw member 100 may be coupled to the rod support member 300 via the detachment-preventing member 200. The screw member 100 may be coupled to the rod support member 300 so as to be rotatable about its longitudinal axis while being prevented from moving in the longitudinal direction.

An external driver (not illustrated) may be detachably coupled to the screw member 100. After the pedicle screw assembly 10 is inserted into the human body, the external driver (not illustrated) may pass through the rod support member 300, the driver support member 400, and the reinforcing member 500, and may be coupled to one end of the screw member 100 in its longitudinal direction.

The detachment-preventing member 200 serves to maintain a coupling state between the screw member 100 and the rod support member 300. The detachment-preventing member 200 is coupled to the screw member 100 and the rod support member 300, respectively.

Specifically, the detachment-preventing member 200 surrounds the different side in the longitudinal direction of the screw member 100, that is, a rear side in the illustrated embodiment, and is coupled to the screw member 100. The different side in the longitudinal direction of the screw member 100 may be received inside the detachment-preventing member 200. In this case, the detachment-preventing member 200 may rotatably receive the different side in the longitudinal direction of the screw member 100.

The detachment-preventing member 200 is coupled to the rod support member 300. The detachment-preventing member 200 is received in a rod support hollow 350 formed inside the rod support member 300. The detachment-preventing member 200 may be disposed so as not to be exposed to the outside of the rod support member 300.

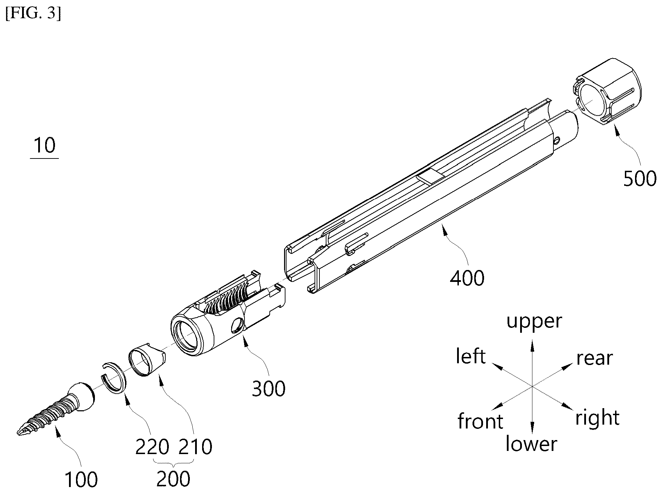

In the embodiment illustrated in FIG. 3, the detachment-preventing member 200 includes a detachment-preventing body 210 and a detachment-preventing ring 220.

The detachment-preventing body 210 rotatably receives a portion of the different side in the longitudinal direction of the screw member 100. The detachment-preventing body 210 is received in the rod support hollow 350 of the rod support member 300 and is supported by an inner surface of a rod support body 310. In an embodiment, the detachment-preventing body 210 may rotatably receive the screw member 100 and may also be rotatably received in the rod support hollow 350.

The detachment-preventing ring 220 rotatably receives another portion of the different side in the longitudinal direction of the screw member 100. In an embodiment, the detachment-preventing ring 220 is formed to have an outer diameter larger than that of the detachment-preventing body 210.

The detachment-preventing ring 220 prevents the detachment-preventing body 210, which is received in the rod support hollow 350, from being separated in an axial direction of the screw member 100, that is, toward a front side in the illustrated embodiment. The detachment-preventing ring 220 supports the detachment-preventing body 210 at one side in the longitudinal direction, that is, on the front side.

The detachment-preventing ring 220 is coupled to the screw member 100. The detachment-preventing ring 220 is positioned closer to one side, that is, the front side in the longitudinal direction of the screw member 100, than the detachment-preventing body 210. The detachment-preventing ring 220 is positioned between a front end of the rod support body 310 and the detachment-preventing body 210.

The detachment-preventing ring 220 is formed in an annular shape, and an opening may be formed at one side thereof. The opening provides a space for allowing the detachment-preventing ring 220 to be elastically deformed so that its outer diameter decreases when being pressed. The detachment-preventing ring 220 may be radially pressed inward and elastically deformed while the screw member 100 passes through it, and may be coupled to the rod support member 300 after the deformation.

When the pressing state is released, the detachment-preventing ring 220 may be elastically deformed radially outward and may come into close contact with the rod support member 300. Accordingly, the detachment-preventing ring 220 can stably support the detachment-preventing body 210 and a rod (not illustrated) positioned at its rear side.

The rod support member 300 serves as a component to which an external rod (not illustrated) is coupled. The rod support member 300 supports the external rod (not illustrated). The external rod (not illustrated) may extend along pedicles or a spine by being supported by a plurality of rod support members 300 respectively provided in a plurality of pedicle screw assemblies 10.

The rod support member 300 is coupled to the screw member 100. Specifically, the rod support member 300 is coupled to the screw member 100 via the detachment-preventing member 200. The rod support member 300 may rotatably receive the detachment-preventing member 200, which rotatably supports the screw member 100.

The rod support member 300 extends in a longitudinal direction of the screw member 100, that is, in a front-rear direction in the illustrated embodiment. One side in the extending direction of the rod support member 300, that is, a front side in the illustrated embodiment, surrounds the screw member 100 and the detachment-preventing member 200 coupled thereto. The other side in the extending direction of the rod support member 300, that is, a rear side in the illustrated embodiment, is coupled to the driver support member 400.

A hollow (that is, a rod support hollow 350 to be described later) is formed inside the rod support member 300. A driver (not illustrated) for operating the screw member 100 may be insertable into and withdrawable from the rod support member 300.

The rod support member 300 is coupled to the driver support member 400. The rod support member 300 may be at least partially received in the driver support member 400. A part of the rod support member 300 may be withdrawn to the outside of the human body together with the driver support member 400.

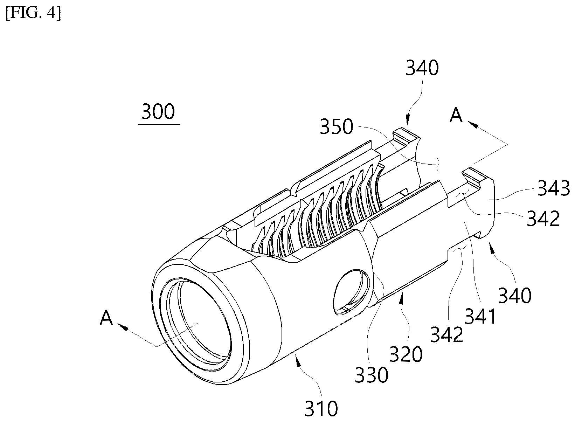

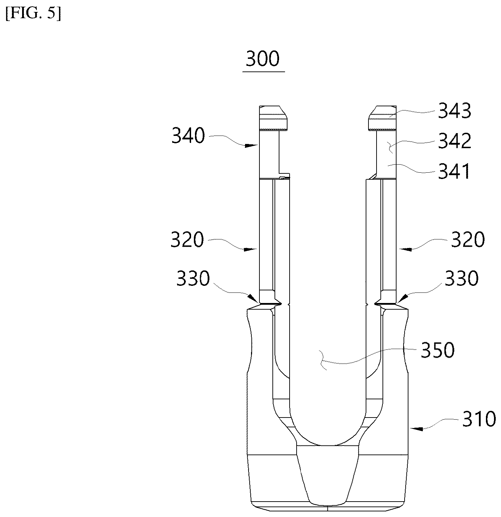

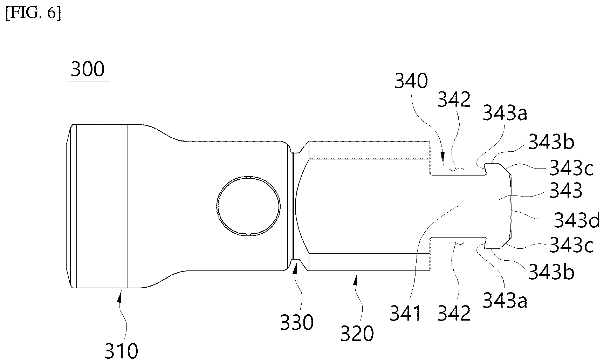

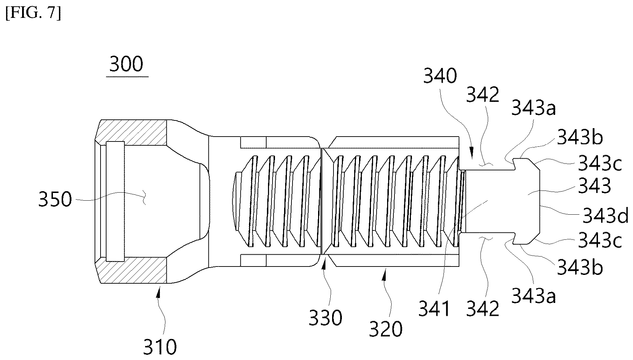

In the embodiments illustrated in FIGS. 4 to 7, the rod support member 300 includes a rod support body 310, a rod support wing 320, a rod connector 330, a support-member coupler 340, and a rod support hollow 350.

The rod support body 310 constitutes a part of an outer shape of the rod support member 300. A space is formed inside the rod support body 310 so that the screw member 100 and the detachment-preventing member 200 can be received therein.

The rod support body 310 may have any shape capable of receiving and supporting the screw member 100 and the detachment-preventing member 200. In the illustrated embodiment, the rod support body 310 has a three-dimensional shape having a front-rear length and an annular cross-section with a hollow formed therein.

The rod support body 310 is coupled to the rod support wing 320. The rod support body 310 is continuous with the rod support wing 320 via a rod connector 330. When the pedicle screw assembly 10 is completely coupled to the pedicle, the rod support body 310 may be separable from the rod support wing 320.

Although no reference numeral is assigned thereto, a thread may be formed on an inner circumferential surface of the rod support body 310. A nut member (not illustrated) for supporting the rod (not illustrated) may be coupled to the thread.

The rod support wing 320 constitutes another part of an outer shape of the rod support member 300. The rod support wing 320 is continuous with an outer circumference of the rod support body 310. One side in the longitudinal direction of the rod support wing 320, that is, a front side in the illustrated embodiment, is continuous with the other side in the longitudinal direction of the rod support body 310, that is, a rear side.

The rod support wing 320 may be continuous with the rod support body 310 via the rod connector 330. As described above, the rod support wing 320 may be separated from the rod support body 310 and withdrawn to the outside of the human body.

The rod support wing 320 is continuous with the support-member coupler 340. One side in the longitudinal direction of the support-member coupler 340, that is, a front side in the illustrated embodiment, is continuous with the different side in the longitudinal direction of the rod support wing 320, that is, a rear side in the illustrated embodiment. The rod support wing 320 supports a rod coupling projection 422, which is received in a coupling groove 342, from the front side.

A plurality of rod support wings 320 may be provided. The plurality of rod support wings 320 may be spaced apart from each other and may be coupled to the rod support body 310 at different positions. In the illustrated embodiment, a pair of rod support wings 320 are provided, and the pair are continuous with rear ends of left and right sides, respectively.

The rod support wing 320 may be formed to partially surround the rod support hollow 350 in a radial direction. In other words, the rod support wing 320 may have an arc-shaped cross-section extending by a smaller length than the rod support body 310.

The rod connector 330 connects the rod support body 310 and the rod support wing 320. In this case, the rod connector 330 may detachably couple the rod support body 310 and the rod support wing 320. For this purpose, the rod connector 330 may be formed to have a smaller thickness than the rod support body 310 or the rod support wing 320.

After coupling between the pedicle screw assembly 10 and the pedicle is completed, the rod support body 310 and the rod support wing 320 may be separated from each other with respect to the rod connector 330, and the rod support wing 320 and other components coupled thereto may be withdrawn to the outside of the human body.

The rod connector 330 is continuous with the rod support body 310 and the rod support wing 320, respectively. In the illustrated embodiment, the rod connector 330 is continuous with a rear end of the rod support body 310 and a front end of the rod support wing 320, respectively.

A plurality of rod couplers 330 may be provided. The plurality of rod couplers 330 may be spaced apart from each other and may separably couple the rod support body 310 and the rod support wing 320 at different positions. In the illustrated embodiment, a pair of rod couplers 330 are provided, and the pair are respectively coupled to the rod support body 310 and the rod support wing 320 positioned at left and right sides.

The support-member coupler 340 serves as a portion at which the rod support member 300 is coupled to the driver support member 400. The support-member coupler 340 may be fixedly coupled to a rod coupler 420 provided in the driver support member 400. In this case, the support-member coupler 340 may be easily coupled to, but inseparable from, the rod coupler 420. In an embodiment, the support-member coupler 340 may be hook-fitted to the rod coupler 420.

Therefore, when the driver support member 400 is withdrawn to the outside of the human body, the support-member coupler 340 and the rod support wing 320 coupled thereto may be separated from the rod support body 310 and withdrawn together to the outside of the human body.

The support-member coupler 340 constitutes a remaining part of an outer shape of the rod support member 300. The support-member coupler 340 is continuous with the rod support wing 320. The support-member coupler 340 extends in a longitudinal direction of the rod support wing 320, that is, in a front-rear direction in the illustrated embodiment.

One end in a longitudinal direction of the support-member coupler 340, that is, a front side in the illustrated embodiment, is continuous with the different end in a longitudinal direction of the rod support wing 320, that is, a rear side in the illustrated embodiment. The different end in the longitudinal direction of the support-member coupler 340, that is, a rear side in the illustrated embodiment, is coupled to a rod coupler 420 of the driver support member 400.

The support-member coupler 340 may have any shape that is continuous with the rod support wing 320 and capable of being coupled to the driver support member 400. In the illustrated embodiment, the support-member coupler 340 has an arc-shaped cross-section corresponding to the shape of the rod support wing 320. In this case, a length of the cross-section of the support-member coupler 340 may be shorter than a length of the cross-section of the rod support body 310 or the rod support wing 320.

A plurality of support-member couplers 340 may be provided. The plurality of support-member couplers 340 may be spaced apart from each other and may be coupled to the rod support wing 320 at different positions. In the illustrated embodiment, a pair of support-member couplers 340 are provided, and the pair are continuous with the pair of rod support wings 320 positioned at left and right sides, respectively.

In the illustrated embodiment, the support-member coupler 340 includes a coupling body 341, a coupling groove 342, and a coupling projection 343.

The coupling body 341 constitutes a part of an outer shape of the support-member coupler 340. The coupling body 341 serves as a portion at which the support-member coupler 340 is connected to the rod support wing 320.

The coupling body 341 has a shape corresponding to the shape of the rod support wing 320, that is, an arc-shaped cross-section, but has a shorter length than a cross-section of the rod support wing 320. In other words, the coupling body 341 is formed to have a shorter length than the rod support wing 320 along an outer circumferential direction.

The coupling body 341 extends in a longitudinal direction of the rod support wing 320, that is, in a front-rear direction in the illustrated embodiment. One end in the longitudinal direction of the coupling body 341, that is, a front end in the illustrated embodiment, is continuous with a rear end of the rod support wing 320. The different end in the longitudinal direction of the coupling body 341, that is, a rear end in the illustrated embodiment, is continuous with the coupling projection 343.

The coupling body 341 partially surrounds the rod support hollow 350. As described above, since a length of a cross-section of the coupling body 341 is shorter than that of the rod support wing 320, so an area by which the coupling body 341 surrounds the rod support hollow 350 may be smaller than an area by which the rod support wing 320 surrounds the rod support hollow 350.

The coupling groove 342 is positioned adjacent to the coupling body 341 along an outer circumferential direction of the rod support member 300. The coupling groove 342 is located between the rod support wing 320 and the coupling projection 343. The coupling groove 342 may be defined as a space surrounded by the rod support wing 320, the coupling body 341, and the coupling projection 343.

The coupling groove 342 partially receives a rod coupler 420 of the driver support member 400. Specifically, the coupling groove 342 receives a rod coupling projection 422. The rod coupling projection 422 received in the coupling groove 342 is not arbitrarily withdrawn from the coupling groove 342 by the coupling projection 343.

The coupling groove 342 may have any shape capable of receiving a rod coupling projection 422 of the rod coupler 420. In the illustrated embodiment, the coupling groove 342 includes a space with a thickness in a radial direction and has an arc-shaped cross-section.

A plurality of coupling grooves 342 may be formed. The plurality of coupling grooves 342 may respectively receive a plurality of rod coupling projections 422 at different positions. In the illustrated embodiment, two pairs of coupling grooves 342 are provided. One pair of coupling grooves 342 are provided in a support-member coupler 340 positioned at a left side, and are arranged to face each other with the coupling body 341 interposed therebetween along an outer circumferential direction. The different pair of coupling grooves 342 are provided in a support-member coupler 340 positioned at a right side, and are arranged to face each other with the coupling body 341 interposed therebetween along the outer circumferential direction.

The coupling projection 343 supports the rod coupling projection 422, which is received in the coupling groove 342, from one side in a longitudinal direction, that is, a rear side in the illustrated embodiment.

The coupling projection 343 supports the rod coupling projection 422 when the driver support member 400 moves to one side in the longitudinal direction, that is, to the rear side in the illustrated embodiment. Accordingly, the support-member coupler 340 and the rod support wing 320 continuous therewith may be separated from the rod support body 310 and withdrawn to the outside of the human body together with the driver support member 400.

The coupling projection 343 may have a shape corresponding to the shape of the rod support wing 320 or the coupling body 341. In the illustrated embodiment, the coupling projection 343 has an arc-shaped cross-section and a thickness in a radial direction.

In this case, the coupling projection 343 may have a cross-sectional length shorter than that of the rod support wing 320. In addition, the coupling projection 343 may have a cross-sectional length longer than that of the coupling body 341. In an embodiment, the coupling projection 343 may have a cross-sectional length equal to a sum of cross-sectional lengths of the coupling body 341 and the pair of coupling grooves 342 surrounding the coupling body 341.

At this time, the coupling projection 343 may be formed to protrude at least partially relative to the coupling body 341 along the radial direction. Therefore, the rod coupling projection 422 received in the coupling groove 342 may be supported by a protruded portion of the coupling projection 343.

In the illustrated embodiment, the coupling projection 343 includes a first projection surface 343a, a second projection surface 343b, a third projection surface 343c, and a fourth projection surface 343d.

The first projection surface 343a constitutes a part of the coupling projection 343. The first projection surface 343a is continuous with a surface in a width direction of the coupling body 341, that is, upper and lower surfaces in the illustrated embodiment. The first projection surface 343a is continuous with the surface in the width direction of the coupling body 341 at a predetermined angle. In an embodiment, the predetermined angle may be an acute angle.

The first projection surface 343a partially surrounds the coupling groove 342. In the illustrated embodiment, the first projection surface 343a surrounds the coupling groove 342 from a rear side.

A plurality of first projection surfaces 343a may be provided. The plurality of first projection surfaces 343a may be continuous with respective surfaces in the width direction of the coupling body 341. In the illustrated embodiment, a pair of first projection surfaces 343a are provided, and the pair are respectively continuous with upper and lower surfaces of the coupling body 341.

The first projection surface 343a is continuous with a second projection surface 343b.

The second projection surface 343b constitutes another part of the coupling projection 343. The second projection surface 343b is continuous with an outer end in a width direction of the first projection surface 343a. The second projection surface 343b is continuous with the first projection surface 343a at a predetermined angle. In an embodiment, the predetermined angle may be an acute angle.

The second projection surface 343b may contact an inner surface of the rod coupler 420 that surrounds a movement groove 421. The second projection surface 343b may be supported by the inner surface of the rod coupler 420. To this end, the second projection surface 343b may extend in the same direction as a side surface of the coupling body 341, that is, in a front-rear direction in the illustrated embodiment.

A plurality of second projection surfaces 343b may be provided. The plurality of second projection surfaces 343b may be continuous with respective first projection surfaces 343a. In the illustrated embodiment, a pair of second projection surfaces 343b are provided, and the pair are respectively continuous with outer ends of the pair of first projection surfaces 343a.

The second projection surface 343b is continuous with a third projection surface 343c.

The third projection surface 343c constitutes still another part of the coupling projection 343. The third projection surface 343c is continuous with one end in a longitudinal direction of the second projection surface 343b, that is, a rear end in the illustrated embodiment. The third projection surface 343c is continuous with the second projection surface 343b at a predetermined angle. In an embodiment, the predetermined angle may be an obtuse angle.

The third projection surface 343c may press the rod coupling projection 422 outward. As will be described later, the rod coupling projection 422 is formed to have a predetermined elasticity. The rod coupling projection 422 may be pressed by the third projection surface 343c, elastically deformed, and store a restoring force.

As insertion of the rod support member 300 into the driver support member 400 proceeds, the rod coupling projection 422 sequentially passes through the third projection surface 343c and the second projection surface 343b, and is received in the coupling groove 342. In this case, the rod coupling projection 422 may be elastically deformed inward by the stored restoring force and received in the coupling groove 342. Accordingly, the rod coupling projection 422 is not arbitrarily withdrawn from the coupling groove 342.

A plurality of third projection surfaces 343c may be provided. The plurality of third projection surfaces 343c may be continuous with respective second projection surfaces 343b. In the illustrated embodiment, a pair of third projection surfaces 343c are provided, and the pair are respectively continuous with rear ends of the pair of second projection surfaces 343b.

The third projection surface 343c is continuous with a fourth projection surface 343d.

The fourth projection surface 343d constitutes a remaining part of the coupling projection 343. The fourth projection surface 343d is continuous with one end in a longitudinal direction of the third projection surface 343c, that is, a rear end in the illustrated embodiment. The fourth projection surface 343d is continuous with the third projection surface 343c at a predetermined angle. In an embodiment, the predetermined angle may be an obtuse angle.

The fourth projection surface 343d may be provided in singular. The singular fourth projection surface 343d may be continuous with a pair of third projection surfaces 343c. In the illustrated embodiment, the fourth projection surface 343d is respectively continuous with rear ends of the pair of third projection surfaces 343c and extends in an up-down direction.

The rod support hollow 350 is a space formed inside the rod support member 300. The rod support hollow 350 is at least partially surrounded by the rod support body 310, the rod support wing 320, and the coupling body 341. In the illustrated embodiment, a front side of the rod support hollow 350 is surrounded by the rod support body 310, a rear side of the rod support hollow 350 is surrounded by the coupling body 341, and a portion between the front and rear sides of the rod support hollow 350 is surrounded by the rod support wing 320.

The rod support hollow 350 receives the detachment-preventing member 200 and the screw member 100 coupled thereto. In an embodiment, the detachment-preventing member 200 may be rotatably received in the rod support hollow 350 around its longitudinal axis.

The rod support hollow 350 receives a driver (not illustrated). The driver (not illustrated) may extend to the screw member 100 through the driver accommodation hollow 440 and the rod support hollow 350. To this end, the rod support hollow 350 may communicate with the driver accommodation hollow 440.

The rod support hollow 350 may have a shape corresponding to the shape of the rod support member 300. In the illustrated embodiment, the rod support hollow 350 is formed as a cylindrical space having a circular cross-section and a height in a front-rear direction.

The driver support member 400 receives and supports a driver (not illustrated) provided from the outside. The driver (not illustrated) may be guided by the driver support member 400 so as not to oscillate in any direction, that is, in a radial direction, and may move along a longitudinal direction to be coupled to the screw member 100.

The driver support member 400 is coupled to the rod support member 300. The driver support member 400 may receive at least a part of the rod support member 300. In an embodiment, the driver support member 400 may be hook-fitted to the rod support member 300. This coupling is achieved through the coupling between the support-member coupler 340 and the rod coupler 420, which will be described later in detail.

The driver support member 400 is coupled to the reinforcing member 500. The driver support member 400, which receives a driver (not illustrated), may be prevented from oscillating in any direction by the reinforcing member 500 and may be maintained in a stable coupling state with the driver (not illustrated).

The driver support member 400 extends in a direction corresponding to an insertion and withdrawal direction of the pedicle screw assembly 10. In the illustrated embodiment, the driver support member 400 extends in a front-rear direction. One side in a longitudinal direction of the driver support member 400, that is, a front side in the illustrated embodiment, is coupled to the rod support member 300. The different side in the longitudinal direction of the driver support member 400, that is, a rear side in the illustrated embodiment, is coupled to the reinforcing member 500.

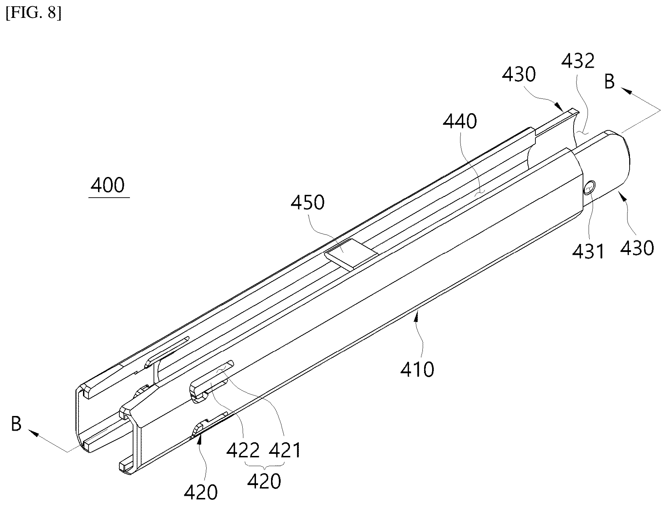

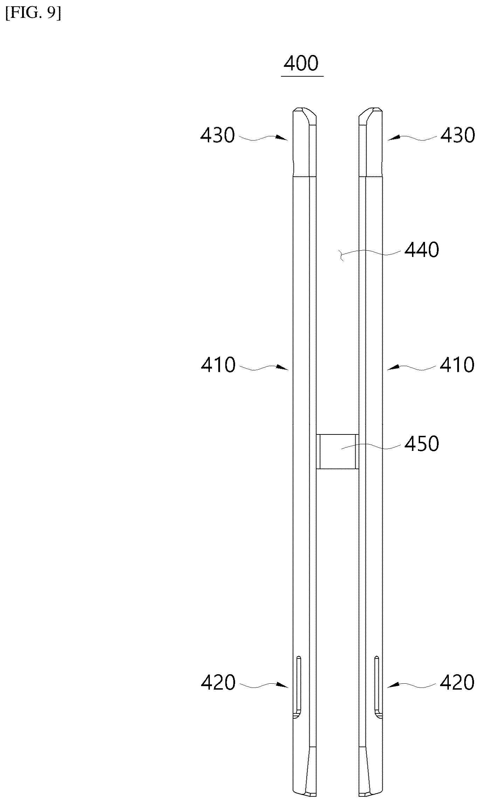

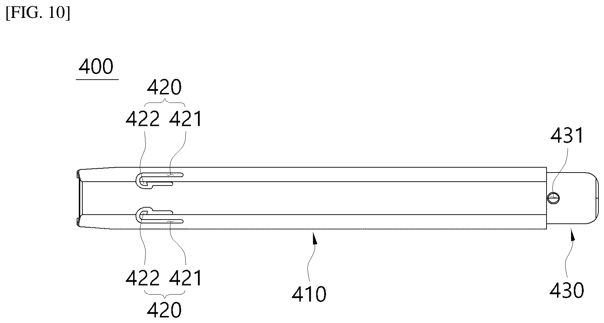

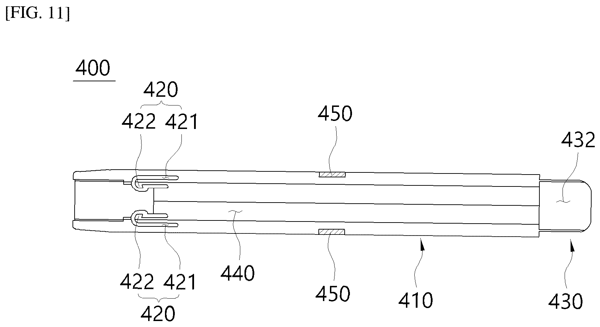

In the embodiment illustrated in FIGS. 8 to 11, the driver support member 400 includes a driver support body 410, a rod coupler 420, a reinforcing-member coupler 430, a driver accommodation hollow 440, and a connection plate 450.

The driver support body 410 constitutes a part of the outer shape of the driver support member 400. The driver support body 410 extends in a longitudinal direction of the driver support member 400, that is, in a front-rear direction in the illustrated embodiment. A space is formed inside the driver support body 410 to accommodate the rod support member 300 or the driver (not illustrated).

The driver support body 410 has a rod coupler 420 formed thereon. The rod coupler 420 is positioned adjacent to one end in a longitudinal direction of the driver support body 410, that is, a front end in the illustrated embodiment.

The driver support body 410 is continuous with the reinforcing-member coupler 430. The different end in the longitudinal direction of the driver support body 410, that is, a rear end in the illustrated embodiment, is continuous with the reinforcing-member coupler 430.

The driver support body 410 at least partially surrounds the driver accommodation hollow 440. In the illustrated embodiment, the driver support body 410 surrounds the driver accommodation hollow 440 in a width direction, that is, in a left-right direction in the illustrated embodiment.

The driver support body 410 is continuous with a connection plate 450. A plurality of driver support bodies 410 may be coupled by the connection plate 450.

A plurality of driver support bodies 410 may be provided. The plurality of driver support bodies 410 may be disposed to face each other with the driver accommodation hollow 440 interposed therebetween. In the illustrated embodiment, a pair of driver support bodies 410 are provided. The pair of driver support bodies 410 are spaced apart from each other in a width direction, that is, on left and right sides with the driver accommodation hollow 440 interposed therebetween. The pair of driver support bodies 410 may be coupled by the connection plate 450.

The rod coupler 420 is a portion where the driver support member 400 and the rod support member 300 are coupled to each other. The rod coupler 420 is coupled to the support-member coupler 340. In an embodiment, the rod coupler 420 may be hook-fitted to the support-member coupler 340. In this embodiment, the rod coupler 420 and the support-member coupler 340 may be easily coupled to each other and may be firmly coupled so as not to be arbitrarily separated.

The rod coupler 420 is positioned in the driver support body 410. A plurality of rod couplers 420 may be formed. The plurality of rod couplers 420 are respectively formed in the plurality of driver support bodies 410 and may be coupled to the plurality of support-member couplers 340, respectively. In the illustrated embodiment, two pairs of rod couplers 420 are provided, and each pair is positioned adjacent to one end in a longitudinal direction of the pair of driver support bodies 410, that is, a front end in the illustrated embodiment.

In this case, the pair of rod couplers 420 may be spaced apart from each other in a height direction of the driver support body 410, that is, in an up-down direction in the illustrated embodiment. The number and arrangement of the rod couplers 420 may vary depending on the number and arrangement of the support-member couplers 340.

In the illustrated embodiment, the rod coupler 420 includes a movement groove 421 and a rod coupling projection 422.

The movement groove 421 provides a space for the rod coupling projection 422 to elastically move. The movement groove 421 is formed to penetrate through a thickness direction of the driver support body 410, that is, in a left-right direction in the illustrated embodiment. The movement groove 421 extends in a longitudinal direction of the driver support body 410, that is, in a front-rear direction in the illustrated embodiment, and has a height in a height direction of the driver support body 410, that is, in an up-down direction in the illustrated embodiment.

In this case, the length and height of the movement groove 421 may each be formed to be greater than the length and height of the rod coupling projection 422, respectively. In addition, the length of the movement groove 421 may be formed to be greater than its height.

Therefore, the rod coupling projection 422 positioned in the movement groove 421 is pressed by the coupling projection 343, easily elastically deformed outward in a height direction, and restored to its original shape by a restoring force, thereby being received in the coupling groove 342.

The rod coupling projection 422 is received in the movement groove 421 and is pressed by the coupling projection 343, thereby being elastically deformed outward in a height direction. The rod coupling projection 422 is elastically deformed and restored inward in the height direction by the restoring force stored therein, and may be received in the coupling groove 342.

The rod coupling projection 422 extends in a longitudinal direction of the driver support body 410, that is, in a front-rear direction in the illustrated embodiment. One end of the rod coupling projection 422 in the longitudinal direction, that is, a front end in the illustrated embodiment, is maintained as a free end. The different end of the rod coupling projection 422 in the longitudinal direction, that is, a rear end in the illustrated embodiment, is continuous with an inner surface of the driver support body 410 surrounding the movement groove 421.

One end of the rod coupling projection 422 in the longitudinal direction, that is, a front end in the illustrated embodiment, is provided with a projection formed to protrude inward in a radial direction. The projection contacts the coupling projection 343, allowing the rod coupling projection 422 to be shape-deformed outward in the radial direction.

The reinforcing-member coupler 430 is a portion where the driver support member 400 and the reinforcing member 500 are coupled to each other. The reinforcing-member coupler 430 may be received in a support-member coupling space 530 of the reinforcing member 500. In an embodiment, the reinforcing-member coupler 430 may be detachably received in the support-member coupling space 530.

The reinforcing-member coupler 430 is continuous with the driver support body 410. The reinforcing-member coupler 430 is continuous with the different end in a longitudinal direction of the driver support body 410, that is, a rear end in the illustrated embodiment.

The reinforcing-member coupler 430 extends in the same direction as the driver support body 410, that is, in a front-rear direction in the illustrated embodiment. One end of the reinforcing-member coupler 430 in the longitudinal direction, that is, a front end in the illustrated embodiment, is continuous with the rear end of the driver support body 410.

A plurality of reinforcing-member couplers 430 may be provided. The plurality of reinforcing-member couplers 430 are respectively continuous with the plurality of driver support bodies 410 and may be respectively coupled to the reinforcing member 500. In the illustrated embodiment, a pair of reinforcing-member couplers 430 are provided and are respectively continuous with the pair of driver support bodies 410. The pair of reinforcing-member couplers 430 may be coupled to the reinforcing member 500 in different positions.

As will be described later, the reinforcing member 500 applies a force directed radially inward to the reinforcing-member coupler 430. Accordingly, the driver support body 410 continuous with the reinforcing-member coupler 430 also receives a force directed radially inward, thereby allowing the accommodated driver (not illustrated) to be stably maintained.

In the illustrated embodiment, the reinforcing-member coupler 430 includes a reinforcing-member receiving groove 431 and a reinforcing-member receiving space 432.

The reinforcing-member receiving groove 431 provides a space for a support-member coupling protrusion 550 provided on the reinforcing member 500 to be inserted. The reinforcing-member receiving groove 431 is recessed on an outer surface of the reinforcing-member coupler 430. The reinforcing-member receiving groove 431 detachably receives the support-member coupling protrusion 550. In an embodiment, the support-member coupling protrusion 550 may be fitted in the reinforcing-member receiving groove 431.

The reinforcing-member receiving groove 431 may be positioned adjacent to one end in a longitudinal direction of the reinforcing-member coupler 430, that is, a front end in the illustrated embodiment. The reinforcing-member receiving groove 431 may be formed to have a circular cross-section.

A plurality of reinforcing-member receiving grooves 431 may be provided. The plurality of reinforcing-member receiving grooves 431 may be respectively formed in the plurality of reinforcing-member couplers 430. In the illustrated embodiment, a pair of reinforcing-member receiving grooves 431 are provided and are respectively formed in the pair of reinforcing-member couplers 430.

The shape, number, and position of the reinforcing-member receiving groove 431 may vary depending on the shape, number, and position of the support-member coupling protrusion *550.*

The reinforcing-member receiving space 432 at least partially accommodates the reinforcing member 500. The reinforcing-member receiving space 432 is surrounded by a pair of reinforcing-member couplers 430. In other words, the reinforcing-member receiving space 432 is a space formed inside the reinforcing-member coupler 430.

An inner portion of the reinforcing member 500, that is, a portion surrounding a driver accommodation hollow 520 in a radial direction, may be accommodated in the reinforcing-member receiving space 432. To this end, the reinforcing-member receiving space 432 may have a shape corresponding to that portion of the reinforcing member 500. In the illustrated embodiment, the reinforcing-member receiving space 432 is formed as a cylindrical shape, having a circular cross-section and a length in a front-rear direction.

The reinforcing-member receiving space 432 communicates with the rod support hollow 350, the driver accommodation hollow 440, and the driver through hollow 520, respectively. The driver (not illustrated) may proceed to the screw member 100 through the reinforcing member 500 accommodated in the reinforcing-member receiving space 432.

In another embodiment (not illustrated), the reinforcing-member coupler 430 is formed as a three-dimensional shape having an annular cross-section with a hollow formed therein and a length in a front-rear direction. In this embodiment, instead of the reinforcing-member receiving groove 431, threads may be formed on at least one of an outer circumference and an inner circumference of the reinforcing-member coupler 430.

In this embodiment, instead of the support-member coupling protrusion 550, threads may be formed on at least one of an inner circumference and an outer circumference of the reinforcing member 500. In this embodiment, the reinforcing member 500 may be screw-coupled to the reinforcing-member coupler 430.

That is, it will be understood that the structure of the reinforcing-member coupler 430 may be modified into any form capable of being coupled to the reinforcing member 500.

The driver accommodation hollow 440 is a space formed inside the driver support member 400. The driver accommodation hollow 440 accommodates a driver (not illustrated). The driver accommodation hollow 440 is defined by being surrounded the driver support body 410 and the connection plate 450. The driver (not illustrated) accommodated in the driver accommodation hollow 440 may be supported in a radial direction by the driver support body 410 and the connection plate 450.

The driver accommodation hollow 440 may have a shape corresponding to the shape of the driver support body 410. In the illustrated embodiment, the driver accommodation hollow 440 is formed as a columnar space having a predetermined cross-section and a length in a front-rear direction.

Each end of the driver accommodation hollow 440 in a longitudinal direction, that is, a front end and a rear end in the illustrated embodiment, is open. The driver accommodation hollow 440 communicates with the rod support hollow 350, the reinforcing-member receiving space 432, and the driver through hollow 520, respectively.

The connection plate 450 couples a plurality of driver support bodies 410. In addition, the connection plate 450 at least partially surrounds the driver accommodation hollow 440.

The connection plate 450 extends between the plurality of driver support bodies 410. In the illustrated embodiment, the connection plate 450 is continuous with a central portion in a longitudinal direction, that is, a front-rear direction, of a pair of driver support bodies 410. The connection plate 450 extends in a width direction of the driver support member 400, that is, in a left-right direction in the illustrated embodiment.

One end of the connection plate 450 in an extending direction, that is, a left end in the illustrated embodiment, is continuous with one of the driver support bodies 410 positioned on the left side. The different end of the connection plate 450 in the extending direction, that is, a right end in the illustrated embodiment, is continuous with another driver support body 410 positioned on the right side.

A plurality of connection plates 450 may be provided. The plurality of connection plates 450 may respectively couple the plurality of driver support bodies 410. In the illustrated embodiment, a pair of connection plates 450 are provided and are respectively positioned at an upper side and a lower side of the pair of driver support bodies 410. The pair of connection plates 450 are positioned to face each other in a height direction, that is, an up-down direction in the illustrated embodiment, with the driver accommodation hollow 440 interposed therebetween.

The reinforcing member 500 is coupled to the driver support member 400 and is configured to prevent the driver support member 400 from being expanded. In other words, the reinforcing member 500 stabilizes the structure of the driver support member 400. By the reinforcing member 500, the structure of the driver support member 400 to which a driver (not illustrated) is coupled can be stably maintained, so that a coupling process between the pedicle screw assembly 10 and a pedicle can be stably maintained.

The reinforcing member 500 is coupled to the driver support member 400. The reinforcing member 500 may be detachably coupled to the reinforcing-member coupler 430. The reinforcing member 500 is disposed to face the screw member 100, the detachment-preventing member 200, and the rod support member 300 with the driver support member 400 interposed therebetween.

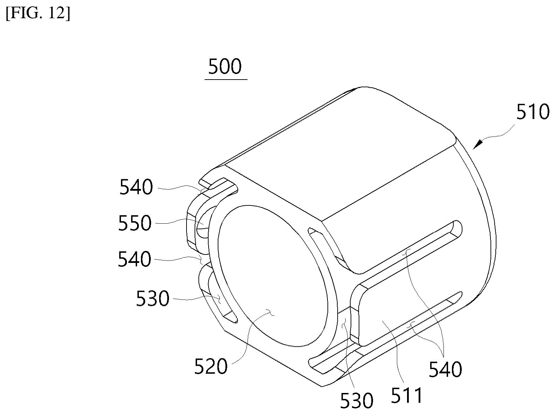

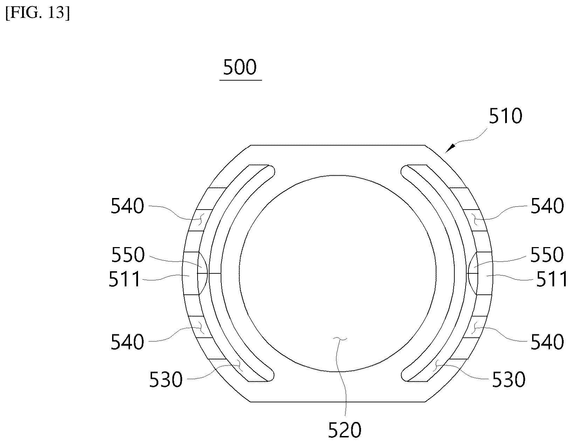

In the embodiments illustrated in FIGS. 12 and 13, the reinforcing member 500 includes a reinforcing body 510, a driver through hollow 520, a support-member coupling space 530, a slitted groove 540, and a support-member coupling protrusion 550.

The reinforcing body 510 forms an outer shape of the reinforcing member 500. Each component of the reinforcing member 500 may be formed in the reinforcing body 510. In the illustrated embodiment, the reinforcing body 510 includes the driver through hollow 520, the support-member coupling space 530, the slitted groove 540, and the support-member coupling protrusion 550.

The reinforcing body 510 is coupled to the reinforcing-member coupler 430. To this end, the reinforcing body 510 may be formed to have a shape corresponding to the shape of the reinforcing-member coupler 430. In the illustrated embodiment, the reinforcing body 510 is formed as a three-dimensional shape having an oblong cross-section and a height in a front-rear direction.

In this case, the reinforcing body 510 may be configured such that an area of a portion protruding outward relative to the driver support body 410 is minimized. Accordingly, the pedicle screw assembly 10 can be easily inserted into or withdrawn from a human body while the reinforcing body 510 is coupled.

The reinforcing body 510 may be divided into a plurality of portions. Specifically, the reinforcing body 510 may include an inner portion positioned inward to surround the driver through hollow 520, and an outer portion positioned outward to surround the support-member coupling space 530 and having the slitted groove 540 and the support-member coupling protrusion 550 formed thereon.

In the illustrated embodiment, the reinforcing body 510 includes a reinforcing wing 511.

The reinforcing wing 511 is defined as a part of an outer portion of the reinforcing body 510. The reinforcing wing 511 is made of a material having a predetermined elasticity and is configured to be elastically deformable along a radial direction of the reinforcing body 510. The reinforcing wing 511 is positioned to partially surround the support-member coupling space 530 from a radially outer side.

The reinforcing wing 511 may surround an outer surface of the reinforcing-member coupler 430. A pair of slitted grooves 540 are positioned along an outer circumferential direction of the reinforcing wing 511 to provide spaces for the reinforcing wing 511 to be elastically deformed and restored. A support-member coupling protrusion 550 is formed to protrude from an inner surface of the reinforcing wing 511.

A plurality of reinforcing wings 511 may be provided. The plurality of reinforcing wings 511 may be coupled to cover the reinforcing-member coupler 430 at different positions. In the illustrated embodiment, a pair of reinforcing wings 511 are provided and are spaced apart from each other in a width direction of the reinforcing member 500, that is, in left and right directions in the illustrated embodiment.

The driver through hollow 520 is a space through which a driver (not illustrated) passes. The driver through hollow 520 is formed to penetrate the reinforcing body 510 in a longitudinal direction, that is, a front-rear direction in the illustrated embodiment. Each end of the driver through hollow 520 in the extending direction, that is, a front end and a rear end in the illustrated embodiment, is open and communicates with the driver accommodation hollow 440 and the outside, respectively.

The driver through hollow 520 is defined by the inner portion of the reinforcing body 510. The shape of the driver through hollow 520 may correspond to the shape of the reinforcing-member coupler 430. In the illustrated embodiment, the driver through hollow 520 is formed as a cylindrical space having a circular cross-section and a length in a front-rear direction.

The support-member coupling space 530 is a space for accommodating the reinforcing-member coupler 430. The support-member coupling space 530 is formed between the inner portion and the outer portion of the reinforcing body 510. That is, the support-member coupling space 530 is defined by the inner portion and the outer portion of the reinforcing body 510. The support-member coupling space 530 is physically separated from the driver through hollow 520 by the inner portion of the reinforcing body 510.

The support-member coupling space 530 may have any shape capable of accommodating the reinforcing-member coupler 430. In the illustrated embodiment, the support-member coupling space 530 is formed as a three-dimensional space having an arc-shaped cross-section and a length in a front-rear direction.

In this case, one end of the support-member coupling space 530 in a longitudinal direction, that is, a front end in the illustrated embodiment, is open to form a passage through which the reinforcing-member coupler 430 can be inserted and withdrawn. The different end of the support-member coupling space 530 in the longitudinal direction, that is, a rear end in the illustrated embodiment, is closed to limit an insertion distance of the reinforcing-member coupler 430.

A plurality of support-member coupling spaces 530 may be formed. The plurality of support-member coupling spaces 530 may be spaced apart from each other and may respectively accommodate the plurality of reinforcing-member couplers 430. In the illustrated embodiment, a pair of support-member coupling spaces 530 are provided and are respectively positioned in a width direction of the reinforcing member 500, that is, in left and right directions in the illustrated embodiment.

The slitted groove 540 provides a space for the reinforcing wing 511 to move in a radial direction. In other words, the slitted groove 540 partially separates the reinforcing wing 511 from an outer portion of the reinforcing body 510 so that the reinforcing wing 511 can be configured to move in a limited manner.

The slitted groove 540 is formed to surround the reinforcing wing 511 along an outer circumferential direction. In the illustrated embodiment, a pair of slitted grooves 540 are provided and are respectively formed at upper and lower sides of the reinforcing wing 511. The pair of slitted grooves 540 are positioned to face each other along the outer circumferential direction of the reinforcing wing 511.

The slitted groove 540 extends in a longitudinal direction of the reinforcing body 510, that is, a front-rear direction in the illustrated embodiment. In this case, one end of the slitted groove 540 in the extending direction, that is, a front end in the illustrated embodiment, is open, and the different end of the slitted groove 540 in the extending direction, that is, a rear end in the illustrated embodiment, is closed. In other words, the slitted groove 540 is formed to have a length shorter than that of the reinforcing body 510.

A plurality of pairs of slitted grooves 540 may be provided. Each pair of slitted grooves 540 may surround a plurality of reinforcing wings 511, respectively. In the illustrated embodiment, two pairs of slitted grooves 540 are provided and are positioned to surround the pair of reinforcing wings 511 positioned at left and right sides along an outer circumferential direction.

The support-member coupling protrusion 550 is detachably received in the reinforcing-member receiving groove 431. The support-member coupling protrusion 550 is formed to protrude radially inward from an inner circumference of the reinforcing wing 511. In this case, the support-member coupling protrusion 550 may be positioned adjacent to one end of the reinforcing wing 511 in a longitudinal direction, that is, a front end in the illustrated embodiment.

When the reinforcing member 500 is coupled to the reinforcing-member coupler 430, the support-member coupling protrusion 550 and the reinforcing wing 511 coupled thereto are pressed radially outward by an outer surface of the reinforcing-member coupler 430. Accordingly, the reinforcing wing 511 is elastically deformed radially outward and stores a restoring force.

When the movement of the reinforcing member 500 proceeds such that the support-member coupling protrusion 550 is received in the reinforcing-member receiving groove 431, the reinforcing wing 511 is elastically restored radially inward by the stored restoring force and covers the outer surface of the reinforcing-member coupler 430. Accordingly, the reinforcing-member coupler 430 and the reinforcing member 500 can be coupled to each other.

The support-member coupling protrusion 550 may have any shape capable of being received in the reinforcing-member receiving groove 431. In the illustrated embodiment, the support-member coupling protrusion 550 is formed to have a circular cross-section and a thickness in a left-right direction, and is formed such that its cross-sectional area decreases along a protruding direction.

A plurality of support-member coupling protrusions 550 may be provided. The plurality of support-member coupling protrusions 550 may be positioned on inner surfaces of the plurality of reinforcing wings 511, respectively. In the illustrated embodiment, a pair of support-member coupling protrusions 550 are provided and are respectively positioned on inner surfaces of the pair of reinforcing wings 511.

The shape, position, number, and arrangement of the support-member coupling protrusions 550 may be changed according to the shape, position, number, and arrangement of the reinforcing-member receiving grooves 431.

Meanwhile, as described above, the reinforcing-member coupler 430 may have any form capable of being coupled to the reinforcing member 500. For example, the reinforcing-member coupler 430 and the reinforcing member 500 may be screw-coupled to each other or may be coupled to each other by a separate fastening member.

In any case, it is sufficient that the reinforcing-member coupler 430 and the reinforcing member 500 are provided to correspond to each other and can be stably coupled to each other.

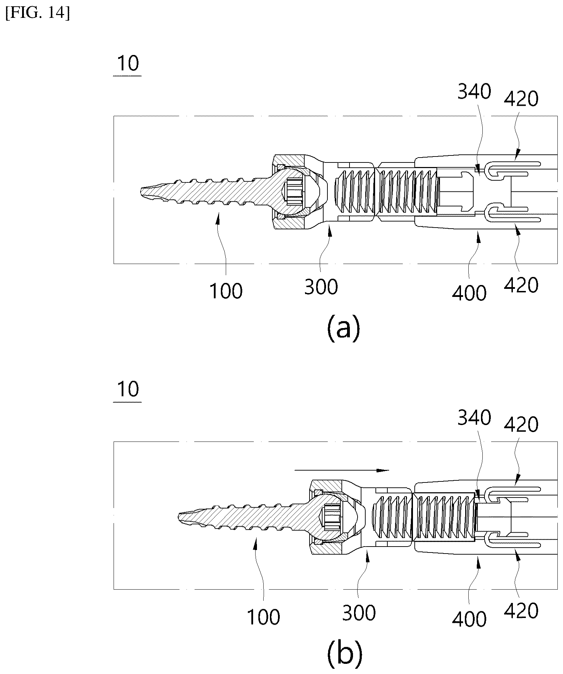

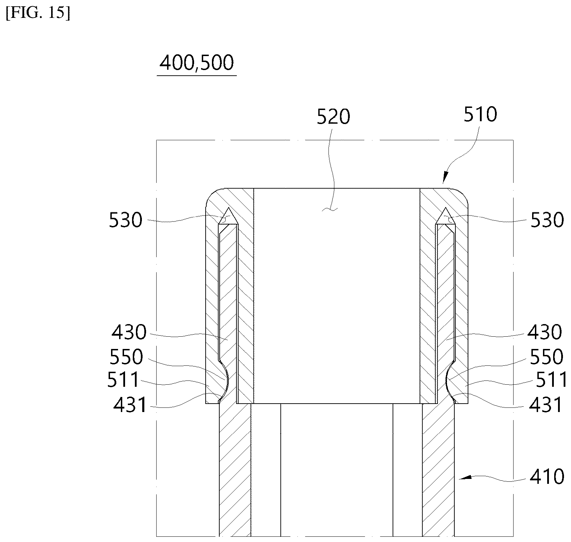

Referring to FIGS. 14 and 15, coupling structures of respective components of the pedicle screw assembly 10 according to an embodiment of the present disclosure are illustrated as examples.

Referring to FIG. 14, a coupling process between a rod support member 300 and a driver support member 400 is illustrated as an example. As described above, the rod support member 300 is at least partially inserted into the driver support member 400 and may be coupled to the driver support member 400 by engagement between a support-member coupler 340 and a rod coupler 420.

As illustrated in part (a) of FIG. 14, the rod support member 300 is moved rearward while being received in the driver accommodation hollow 440 at the different side in the longitudinal direction, that is, at a rear side of the rod support member 300.

As the movement of the rod support member 300 proceeds, a coupling projection 343 of the support-member coupler 340 comes into contact with a rod coupling projection 422. A projection protruding inward is formed at an end of the rod coupling projection 422, and the coupling projection 343 presses and moves the projection.

In this case, a third projection surface 343c, which is a portion initially contacting the projection, extends inwardly and is inclined along the movement direction of the rod support member 300. Accordingly, the rod support member 300 can smoothly move while the third projection surface 343c and a second projection surface 343b, which extends at an obtuse angle thereto, are in contact with the projection.

As illustrated in part (b) of FIG. 14, when the rod support member 300 is sufficiently moved (that is, sufficiently inserted into the driver support member 400), the projection is spaced apart from the second projection surface 343b and is received in the coupling groove 342.

In this case, a first projection surface 343a surrounding the coupling groove 342 extends inwardly and is inclined along the same direction as the third projection surface 343c, that is, along the movement direction of the rod support member 300. Accordingly, even when the rod support member 300 is moved in a direction in which it is separated from the driver support member 400, the rod coupling projection 422 can be supported by the first projection surface 343a.

As a result, the rod support member 300 and the driver support member 400 can be easily coupled to each other, and their coupled state can be stably maintained.

As illustrated in FIG. 15, a state in which the driver support member 400 and the reinforcing member 500 are coupled to each other is illustrated as an example. As described above, the reinforcing member 500 receives the reinforcing-member coupler 430 and can be coupled to the driver support member 400.

As the reinforcing member 500 moves toward the driver support member 400, the reinforcing-member coupler 430 is inserted into the support-member coupling space 530. In this case, the support-member coupling protrusion 550 and the reinforcing wing 511 coupled thereto are pressed radially outward by the reinforcing-member coupler 430, thereby being elastically deformed and storing restoring force.

As the movement of the reinforcing member 500 proceeds, the support-member coupling protrusion 550 is received in the reinforcing-member receiving groove 431. Accordingly, the reinforcing wing 511 is elastically deformed radially inward by the restoring force stored therein, thereby covering the reinforcing-member coupler 430.

Therefore, the driver support member 400 and the reinforcing member 500 can also be easily coupled to each other, and their coupled state can be stably maintained.

As a result, in the pedicle screw assembly 10 according to an embodiment of the present disclosure, the reinforcing member 500 can stabilize the structure of the driver support member 400 and a driver (not illustrated) coupled thereto. At the same time, the reinforcing member 500 can be easily and firmly coupled to the driver support member 400. Accordingly, the overall volume of the pedicle screw assembly 10 can be simplified, and the assembly can be precisely positioned at a target region, thereby minimizing an invasive site.

Although exemplary embodiments of the present disclosure have been described above, the scope of the present disclosure is not limited to the embodiments set forth herein. Those skilled in the art who understand the spirit of the present disclosure may readily propose other embodiments by adding, modifying, deleting, or supplementing components within the scope of the present disclosure, and such embodiments should also be regarded as falling within the scope of the present disclosure.

| | | | |
|---|---|---|---|
| 10: | pedicle screw assembly | 100: | screw member |
| 200: | detachment-preventing member | 210: | detachment-preventing body |
| 220: | detachment-preventing ring | 300: | rod support member |
| 310: | rod support body | 320: | rod support wing |
| 330: | rod connector | 340: | support-member coupler |
| 341: | coupling body | 342: | coupling groove |
| 343: | coupling projection | 343a: | first projection surface |
| 343b: | second projection surface | 343c: | third projection surface |
| 343d: | fourth projection surface | 350: | rod support hollow |
| 400: | driver support member | 410: | driver support body |
| 420: | rod coupler | 421: | movement groove |
| 422: | rod coupling projection | 430: | reinforcing-member coupler |
| 431: | reinforcing-member receiving groove space | 432: | reinforcing-member receiving |
| 440: | driver accommodation hollow | 450: | connecting plate |
| 500: | reinforcing member | 510: | reinforcing body |
| 511: | reinforcing wing | 520: | driver through hollow |
| 530: | support-member coupling space | 540: | slitted groove |
| 550: | support-member coupling protrusion | | |

## Claims

1. A pedicle screw assembly comprising:
a screw member configured to be coupled to a pedicle;
a rod support member configured to rotatably support the screw member;
a driver support member extending in one direction, one side of the driver support member being coupled to the rod support member; and
a reinforcing member coupled to a different side of the driver support member, the reinforcing member being configured to press the driver support member radially inward,
wherein the driver support member includes:
a driver support body extending in the one direction, one side of the driver support body in the extending direction being coupled to the rod support member; and
a reinforcing-member coupler extending in the one direction, the reinforcing-member coupler being continuous with a different side of the driver support body in the extending direction and being coupled to the reinforcing member.

2. The pedicle screw assembly of claim 1, wherein the reinforcing member includes:
a reinforcing body configured to surround the reinforcing-member coupling portion and to be coupled to the driver support member; and
a support-member coupling space formed inside the reinforcing body, the support-member coupling space being open on one side facing the reinforcing member so as to detachably receive the reinforcing-member coupler.

3. The pedicle screw assembly of claim 2, wherein the driver support body includes a pair of driver support bodies, the pair being spaced apart from each other in a different direction;
the reinforcing-member coupler includes a pair of reinforcing-member couplers, the pair being respectively continuous with the pair of driver support bodies; and
the support-member coupling space includes a pair of support-member coupling spaces, the pair being spaced apart from each other in the different direction and respectively receiving the pair of reinforcing-member couplers.

4. The pedicle screw assembly of claim 2, wherein the reinforcing-member coupler includes a reinforcing-member receiving groove, the reinforcing-member receiving groove being recessed on an outer surface of the reinforcing-member coupler, and
wherein reinforcing member includes a support-member coupling protrusion protruding from an inner surface surrounding the support-member coupling space so as to be received in the reinforcing-member receiving groove.

5. The pedicle screw assembly of claim 2, wherein the reinforcing member is divided into one portion positioned inward and a different portion positioned outward along a radial direction with respect to the support-member coupling space, and
wherein the different portion of the reinforcing member includes a slitted groove, the slitted groove extending along a height direction of the reinforcing member and penetrating through a thickness direction of the different portion.

6. The pedicle screw assembly of claim 5, wherein the reinforcing member includes a pair of slitted grooves, the pair being spaced apart from each other along an outer circumference of the different portion of the reinforcing member, and
wherein the reinforcing body includes a reinforcing wing positioned between the pair of slitted grooves, the reinforcing wing being configured to be elastically deformable radially outward when pressed by the reinforcing-member coupler.

7. The pedicle screw assembly of claim 6, wherein the reinforcing member includes a support-member coupling protrusion, the support-member coupling protrusion protruding from an inner surface of the reinforcing wing so as to contact the reinforcing-member coupler.

8. The pedicle screw assembly of claim 1, wherein a thread is formed on an outer circumferential surface of the reinforcing-member coupler,
a thread is formed on an inner circumferential surface of the reinforcing member,
such that the reinforcing-member coupler and the reinforcing member are screw-coupled to each other.

9. The pedicle screw assembly of claim 1, wherein the rod support member includes a support-member coupler received in the driver support member and coupled thereto, and
the driver support member includes a rod coupler positioned at one side of the driver support body and coupled to the support-member coupler.

10. The pedicle screw assembly of claim 9, wherein the support member coupler and the rod coupler are hook-fitted to each other.

11. The pedicle screw assembly of claim 9, wherein the rod coupler includes:
a movement groove configured to penetrate through a thickness direction of the driver support body and to extend along a longitudinal direction thereof; and
a rod coupling projection disposed in the movement groove and extending in the same direction as the movement groove, one end thereof facing the rod support member, being formed as a free end, and the other end thereof facing the reinforcing member, being coupled to the driver support body.

12. The pedicle screw assembly of claim 11, wherein the support-member coupler includes:
a coupling body extending in the same direction as the rod coupling projection;
a coupling groove recessed at each edge in a width direction of the coupling body; and
a coupling projection positioned at one end of the coupling body facing the driver support member, the coupling projection being configured to have a width greater than that of the coupling body.

13. The pedicle screw assembly of claim 12, wherein the rod coupling projection includes a projection formed at one end thereof and protruding facing the rod support member, and
the rod coupling projection is received in the coupling groove after the projection contacts the coupling projection and is elastically deformed outward.

14. The pedicle screw assembly of claim 12, wherein the coupling projection includes:
a first projection surface positioned farthest from the driver support member and extending outward from an outer surface of the coupling body;
a second projection surface continuous with the first projection surface and extending inward and inclined toward the driver support member; and
a third projection surface continuous with the second projection surface and extending inward and inclined toward the driver support member,
wherein the rod coupling projection is received in the coupling groove after being shape-deformed as it sequentially contacts the third projection surface and the second projection surface.
